# EUROPEAN PATENT SPECIFICATION

(11) **EP 3 911 952 B1**
(45) Date of publication and mention of the grant of the patent: **30.04.2025**
(21) Application number: 20904343.9
(22) Date of filing: 14.12.2020
(51) Int. Cl.: G01N 33/543, G01N 33/569

(54) **MANNHEIMIA HAEMOLYTICA SEROTYPE A1 LATERAL FLOW IMMUNOCHROMATOGRAPHY DIAGNOSIS KIT**
LATERALFLUSS-IMMUNCHROMATOGRAFIE-DIAGNOSEKIT DES MANNHEIMIA HAEMOLYTICA-SEROTYPS A1
TROUSSE DE DIAGNOSTIC DE MANNHEIMIA HAEMOLYTICA SÉROTYPE A1 PAR IMMUNOCHROMATOGRAPHY À FLUX LATÉRAL

(30) Priority: 26.12.2019 TR 201921709
(43) Date of publication of application: 24.11.2021
(73) Proprietor: T.C. Erciyes Universitesi, 38039 Talas/Kayseri (TR)
(72) Inventor: GUNES, Vehbi, Kayseri (TR); KELES, Ihsan, Kayseri (TR); CITIL, Mehmet, Kayseri (TR); GUMUSSOY, Semih, Kayseri (TR); ONMAZ, Ali Cesur, Kayseri (TR); EKINCI, Gencay, Kayseri 38090 (TR)
(74) Representative: Yalçiner Patent and Consulting Ltd.
(86) International application number: PCT/TR2020/051291
(87) International publication number: WO 2021/133328

(56) References cited:
- CN-A- 109 239 341
- KUMAR JYOTI ET AL: "Rapid detection of Mannheimia haemolytica in lung tissues of sheep and from bacterial culture", VETERINARY WORLD, 8(9), 1 August 2015 (2015-08-01), pages 1073 - 1077, XP055897039, Retrieved from the Internet <URL:https://www.ncbi.nlm.nih.gov/pmc/articles/PMC4774775/pdf/VetWorld-8-1073.pdf> [retrieved on 20220302], DOI: 10.14202/vetworld.2015.1073-1077
- ANONYMOUS: "Handbook - Bio-T kit Mannheimia haemolytica & Pasteurella multocida Cat. N° BIOTK051", 1 January 2019 (2019-01-01), pages 1 - 16, XP055897040, Retrieved from the Internet <URL:https://biosellal.com/fr/index.php?controller=attachment&id_attachment=124> [retrieved on 20220302]
- RIDLEY JENNIFER LYNN: "Purification and Analysis of an 18KD Outer Membrane Protein of Mannheimia (Pasteurella) Haemolytica", CHANCELLOR'S HONORS PROGRAM PROJECTS, UNIVERSITY OF TENNESSEE, KNOXVILLE, USA, 1 January 2001 (2001-01-01), pages 1 - 49, XP055897163, Retrieved from the Internet <URL:https://trace.tennessee.edu/utk_chanhonoproj/491/> [retrieved on 20220303]
- HOUNSOME JONATHAN D. A. ET AL: "Outer Membrane Protein A of Bovine and Ovine Isolates of Mannheimia haemolytica Is Surface Exposed and Contains Host Species-Specific Epitopes", INFECTION AND IMMUNITY, 1 November 2011 (2011-11-01), pages 4332 - 4341, XP055897182, Retrieved from the Internet <URL:https://www.ncbi.nlm.nih.gov/pmc/articles/PMC3257919/pdf/zii4332.pdf> [retrieved on 20220303], DOI: 10.1128/IAI.05469-11
- BAHADIR ELIF BURCU ET AL: "Lateral flow assays: Principles, designs and labels", TRAC TRENDS IN ANALYTICAL CHEMISTRY, ELSEVIER, AMSTERDAM, NL, vol. 82, 16 June 2016 (2016-06-16), pages 286 - 306, XP029706315, ISSN: 0165-9936, DOI: 10.1016/J.TRAC.2016.06.006
- TEL, OSMAN YASAR; KESKIN, OKTAY; ERDENLIG GÜRBILEK, SEVIL: "Developing Lateral Flow Assay Based Rapid Diagnosis Kit For Serologic Diagnosis of Cattle Brucellosis", VAN VETERINARY JOURNAL, vol. 29, no. 1, 10 October 2017 (2017-10-10), Turkey, pages 13 - 16, XP009529699, ISSN: 2149-3359
- BAHADIR ELIF BURCU; SEZGINTÜRK MUSTAFA KEMAL: "Lateral flow assays: Principles, designs and labels", TRAC TRENDS IN ANALYTICAL CHEMISTRY, vol. 82, 16 June 2016 (2016-06-16), AMSTERDAM, NL, pages 286 - 306, XP029706315, ISSN: 0165-9936, DOI: 10.1016/j.trac.2016.06.006

## Description

### Technical Field

The invention is related to the Lateral Flow analysis systems which enable the on-site detection of *Mannheimia haemolytica* (*M. haemolytica*), which is an important bacterial etiological agent causing respiratory system disease in cattle. Nowadays, Lateral Flow Immunochromatography (LFI) kits are used more frequently in the diagnosis of some infectious diseases and the detection of biological indicators in Medical and Veterinary Practices. In addition, LFI kits are used in food, beverage, pharmaceutical and biological sectors.

### Prior Art

The lateral flow technology (LFT) was developed for the first time for humans, by means of the production of fast birth control kits which determine the human chorionic gonadotropin (HCG) as the qualifier. With the development and production of fast test kits that gained momentum rapidly after 1980s, they were able to be used in many fields. Lateral Flow Immunochromatography technology is based on the application of suitable primary and secondary antibodies on a membrane which will provide fluid flow at a particular speed and on the sandwich ELISA model of the antigen antibody complexes received from the fiber membrane that comprises the conjugated antibodies.

Use of these LFI kits in the Veterinary Practice is not as common as in Medical Practice. Studies on developing such kits still continue in some countries (such as USA, South Korea) in Veterinary Practice. However, all of the kits that are used in Turkey, for farm animals or in pet clinics and for the determination of some contagious animal diseases are being outsourced.

There are foreign companies that produce the kits which determine different animal diseases in the Veterinary Practice (Viral, bacterial agents of calf diarrhea, viral and parasitic agents of cats and dogs, foot and mouth disease virus in cattle, BVD, Tuberculosis and Brucella agents). However, kits determining the respiratory system agents are quite limited. *Mannheimia haemolytica,* which is one of the primary bacterial diseases of the respiratory system, is a bacterial agent that causes respiratory system infection in cattle. It causes contagious bovine pneumonia. There are A and T biotypes of the agent. In addition, these biotypes have numerically named sub-serotypes.

The most common serotype in the world and Turkey is A1 and it is one of the important problem of the countries which import live cattle. It causes severe respiratory distress and death within a week at the region where the cattle are transported. Therefore, it causes an important economic loss. As a result of pneumonia caused by *M. haemolytica* needs to be diagnosed at a very early stage. It requires treatment with antibiotics and the mortality rate of it needs to be reduced in the entire world. To achieve this, early diagnosis is required because the chance of treatment decreases significantly in advanced cases and the animals transmit the agent to other animals, so, this lead to higher mortality rates.

Said bacteria resides in the nasal and pharyngeal cavities of the animals as saprophytes, and then it descends to the lower parts of the respiratory system as a result of the suppression of the immune system and it may cause pneumonia and sepsis. However, the number of foreign companies that produce the fast diagnosis kit of the *Mannheimia haemolytica,* which enables the diagnosis of this disease and thereby prevents it from rapidly spreading are not adequate.

Previously, the blood of the deceased animal and the internal organs, the lungs being in the first place, are sent to the laboratory for the diagnosis of *Mannheimia haemolytica,* as a long and tedious method. The diagnosis is made in the laboratory with the following methods of analysis.

### Bacterioscopy

Since the microorganism is found everywhere in the animal in acute cases, preparations are prepared from blood taken from the heart, edema fluids, tissues taken from the spleen, and liver, and bone marrow, and these samples are stained with Giemsa and bipolar microorganisms are sought in the smear. In chronic cases, microorganisms are localized mostly in the organs. A definite diagnosis cannot be reached by observing bipolar microorganisms in the smear samples.

### Culture

Blood agar, serum agar or special agars are seeded with morbid substances. They are left at 37°C for 24-48 hours. The colony morphologies, microscopic morphologies and biochemical characteristics of the reproduced microorganisms are examined.

### Animal Testing

The mice and pigeons are injected and inoculated with the morbid materials that were sent and isolated if necessary from microorganisms. The animals die within 24-48 hours with virulent strain. Smear and seeding are made using the blood of the dead animals and the diagnosis is confirmed.

In the previous technique, some disadvantages are present; such as being able to diagnose *Mannheimia haemolytica* only in the laboratory, that it takes time and has high diagnosis costs and it requires experienced personnel.

Using the previous methods leads to loss of time and application of unnecessary treatment. In addition, the elapsed time can enhance the spread of the disease. Therefore, developing a fast and accurate diagnosis method is needed.

Kumar Jyoti ET AL discloses a rapid detection of *Mannheimia haemolytica* A1 bacteria via PCR detection. In this document use of a lateral flow immunochromatography device using specific antibodies for its detection is not disclosed.

Anonymous: "Handbook - Bio-T kit *Mannheimia haemolytica & Pasteurella multocida* Cat. N° BIOTK051 discloses kit for the detection of M. haemolytica by real-time PCR. It does not disclose the use of a lateral flow immunochromatography device using specific antibodies for its detection.

Ridley Jennifer Lynn describes (abstract) the development of anti-OmpA 1 antibodies (27-1-22 and 27-1) directed to *M. Haemolytica.* Thus, antibodies directed to *Mannheimia haemolytica* Serotype A 1 capsule protein have been described in the art, but they have not been employed in a test strip.

Similarly, Hounsome Jonathan D. A. ET AL describes anti-OmpA 1 but fails to disclose its use in a lateral flow test strip.

BAHADIR ELiF BURCU ET AL discloses a general prior art document on the use of lateral flow assays.

### Brief Description and Aims of the Invention

The present invention is related to Lateral Flow analysis systems which enable the detection of *Mannheimia haemolytica* bacteria on-site, as defined by the claims.

With the invention, direct and early diagnosis of the agent is possible in on-site due to the determination of serotype A1 with specific monoclonal antibodies of the outer membrane (capsule) antigenic protein.

By means of the lateral flow analysis system of the invention, the etiological diagnosis of the animals respiratory system problem can be made within maximum 30 minutes by blood, serum/plasma and deep nasal swap samples taken from the animals. At the same time, it is a rather reliable method in the diagnosis of the disease due to the high bonding capability of the specific group of the antigen (epitope) with the specific region of the antibody (paratope) (high affinity and specificity over 90%).

The Lateral Flow Immunochromatography diagnosis kit can process smaller volumes from more than one sampling type. The samples to be taken are blood serum and bronchoalveolar lavage fluid in the case of septicemia (bloodstream infection). At least 100 microliters are required as the sample volume. It prevents the development of antibiotic resistance with the reduction of unnecessary drug usage due to the accurate diagnosis in a short period of time such as 30 minutes.

One of the innovations put forth by the diagnosis kit of the Lateral Flow Immunochromatography of the invention is the use of monoclonal antibodies produced from two different clones (clone no: 22D4F1 for conjugation, clone: 19A10E9 on the test line) for the first time against the SA1 capsule antigenic peptide of *M. haemolytica* (2 mg/ml). There is only one specific bonding region (paratope) for each monoclonal antibody against the specific antigen. When a monoclonal antibody specific binds to the antigen, another antigen cannot bind with the specific region of that antibody. Therefore, if the target antigen binds with the monoclonal antibody in the conjugation pad in the diagnosis kit of the invention, the same clone in the test line will not bond with the antibody. Therefore, the line which should be determined on the test line will not be seen and the test will not have worked.

However, since different clone antibodies used in the invention have different specific bounding regions (paratope) that can be bound to different epitopes of the same antigen, they will bind to different epitopes of the same antigen. Therefore, if the target antigen is found in the sample, both the antibody in the conjugate and the antibody in the test line may bind to the target antigen as in the sandwich ELISA method. The invention can be distinguished from the prior art as it enables diagnosis with high affinity and specificity as it is used separately with the lateral flow test kit, on the lines of the different clone monoclonal antibodies produced in rats, and the conjugation pad.

Contrary to the difficult and prolonged applications of the prior techniques, since it requires minimum number of steps and minimum interpretation by a specialist, it provides ease of use for the user and does not require technical knowledge and specialty. Thereby, it reduces the diagnosis cost of the disease.

It provides an economic gain for the producer by ensuring direct drug application against the agent quickly as a result of the diagnosis made with the invention. It can also be integrated with integrated electronic reader systems and information systems.

The diagnosis kit of the invention with Lateral Flow Immunochromatography of the *Mannheimia haemolytica* bacteria can be easily scaled for high volume production. At first, all of the reagents, dispersions and necessary solutions are used in liquid state, however, due to the application of the liquid solutions of the monoclonal antibody conjugated with colloidal gold and antibodies in the test and control lines, which are created as the final products, on the nitrocellulose based membranes, drying of their liquid parts, and the antibodies attaching to these membranes as solid phase, a dry final product is obtained. Since the responsive activity of the antibodies which were attached on the membrane and purified from the moisture is 1-2 years, the kits produced with the invention have a stable shelf life of 12-24 months without cooling.

### Description of the Illustrative Figures

**Figure 1****:** Finding the Optimal mAb Concentration by Measuring the Optic Densities of the Conjugates Having Different Concentrations
**Figure 2****:** *Mannheimia haemolytica* Serotype A1 Lateral Flow Immunochromatography Diagnosis Kit

### Description of the Components and Parts of the Invention

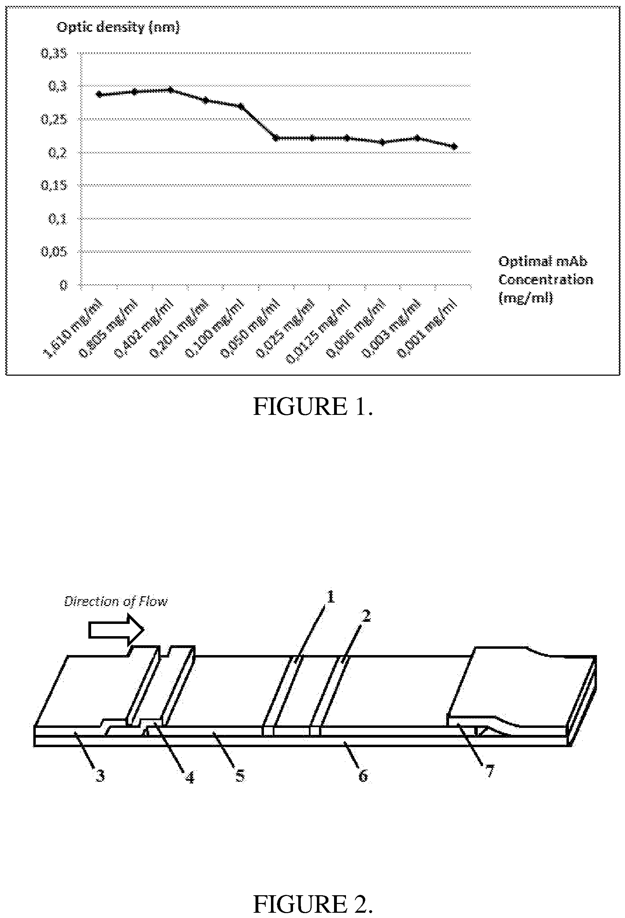

The definitions of the parts that form the *Mannheimia haemolytica* Serotype A1 Lateral Flow Immunochromatography Diagnosis Kit of Figure 2 are given below.
**1:** Test Line *(Mannheimia haemolytica* Serotype A1 Capsule Protein Monoclonal Antibody (mAb), clone no:19A10E9)
**2:** Control Line (Secondary Antibody (Goat Anti-Rat IgG)
**3:** Sample Pad
**4:** Conjugation Pad (Colloidal gold nanoparticle (40 nm) *+ Mannheimia haemolytica* Serotype A1 Capsule Protein Monoclonal Antibody (mAb), clone no: 22D4F1)
**5:** Nitrocellulose Membrane
**6:** Support Card
**7:** Waste Pad

### Detailed Description of the Invention

### Dialysis of the Antibodies

*Mannheimia haemolytica* Serotype A1 capsule protein monoclonal antibody (Monoclonal antibody (mAb) against *Mannheimia haemolytica* A1 capsule protein [Rat antiserotype specific antigen 1 mAb]) (1,610 mg/ml) is dialyzed at +4 ⁰C overnight in 10mM Sodium Phosphate. After the dialysis, it is stored at -20 ⁰C in 1 ml aliquots.

### Optimization

With the purpose of finding, the optimal mAb concentration for conjugation, serial dilutions (½, ¼, 1/8, 1/16, 1/32, 1/68) are made from the stock solution (1,610 mg/ml) and mixed with colloidal gold (DCN, 40 nm) having different pH (pH 8, pH 8,5 and pH 9,0). The colloidal gold enables the covalent binding of the antibodies onto the surface of the particles and therefore the antibodies are biologically marked, and a red line is shown on the Test Line (1). The optic density of the conjugates is measured on 540 mm wavelength and the ideal working concentration and pH is found. Optimum concentration is set to: 0,06 mg/ml (can be between 0,06-0,1 mg/ml), and optimum pH is adjusted to: 9,0.

In Figure 1, finding the saturation point of the conjugate [gold nanoparticle (40 nm) and *Mannheimia haemolytica* Serotype A1 capsule protein monoclonal antibody mixture] by scanning the optic densities at 450 nm wavelength in order to find the most ideal concentration and optimal pH. The point on the graphic where there is diffraction and the line begins to stabilize gives the most ideal monoclonal antibody concentration for conjugation. Therefore, unnecessary use is prevented by using monoclonal antibody having the ideal concentration and the antibodies are used more economically.

### Adjusting the Colloidal Gold Nanoparticle pH

For conjugation, pH 0.2 M Potassium Chloride (+10-30 µl KCl) is added to the colloidal gold nanoparticle (DCN, Colloidal Gold, 0.5L, 40 nm) and the pH is set to 8-9. It is stored at room temperature in dark environment by being wrapping with aluminum foil to be used immediately. Cellulose nanoparticles, latex and colloidal carbon can be used as alternative marker/colorant molecules,

### Conjugation Process

Within the conjugate, monoclonal antibody (0,06 mg/ml, *Mannheimia haemolytica* Serotype A1 Capsule Protein Monoclonal Antibody, Clone no: 22D4F1) (100 µl) 10% by volume and Colloidal Gold Nanoparticle (pH:8-9) (900 µl ) 90% by volume are mixed at room temperature for 15 minutes and the conjugate is prepared.

The conjugate is a dual complex which results from the antibody covalently binding onto the gold nanoparticle surface being composed of a gold nanoparticle and monoclonal antibody. This complex enables the antibody to be visible. The gold nanoparticle which gives the color red advances to the Test Line with the fluid sample after it exhibits binding with the analyte specific monoclonal antibody. Another clone in the test line binds with the antibody and creates a triple complex constituted from target antigen, gold nanoparticle and antibody. Thereby, a visible line appears and the presence of the target analyte is detected.

### Blocking Process

100 µl conjugate block buffer (50 mM Borate, %10 Bovine Serum Albumin (BSA), pH: 9.0) is added to the Colloidal Gold Nanoparticle and *Mannheimia haemolytica* Serotype A1 Capsule Protein mAb, Clone no: 22D4F1 conjugate. It is stirred at room temperature for 30 minutes. This solution is transferred to 1,5 ml eppendorf tubes.

It is centrifuged for 30 minutes at +4 ⁰C as 14.000 g. The supernatant (supernate) is aspirated and discarded with care without damaging the pellet and the process is repeated for a few times in the tubes in which pellets are not formed at the bottom. 750 µl diluent buffer (50 mM Borate, %1 BSA, pH: 9.0) is added on the pellet. It is stirred softly and homogenized.

### Impregnation of the Conjugate by the Conjugation Pad (4)

The colloidal gold nanoparticle and *Mannheimia haemolytica* Serotype A1 capsule protein mAb conjugate is sprayed onto the Conjugation Pad (4) with an automatic pipette such that 6-8 µl will be sprayed for every 1 cm. It is dried in the drying oven for 2 hours at 37 ⁰C. The conjugation pad (4) is a pad composed of glass fiber on which the antibodies are temporarily fixed, containing pores to which nanoparticles can attach.

### Nitrocellulose Membrane (5)

Nitrocellulose membrane (5) is a membrane having a nitrocellulose structure with pores on the same and which provides for the antibody and gold nanoparticles to attach onto the membrane surface, enabling the movement of the fluid sample at a particular speed with the capillary flow force, on which the test (1) and control lines (2) are applied.

### Application of the Test Line (1) onto the Nitrocellulose Membrane

Application on the nitrocellulose membrane is carried out such that 0,1-0,5 µg *Mannheimia haemolytica* Serotype A1 mAb (0,06 mg/ml, Clone: 19A10E9) is spread on 1 cm of the test line (1). It is dried by incubating at room temperature for 1 night.

Test Line (1) is the line on the nitrocellulose where the target analyte found in 0,1-0,5 µg *Mannheimia haemolytica* Serotype A1 capsule protein mAb (0,06 mg/ml, Clone: 19A10E9) as the capturing antibody at 1 cm of the same.

### Application of the Control Line (2) on the Nitrocellulose Membrane

It is applied on the nitrocellulose membrane such that 0,1-0,5 µg Secondary Antibody *(Mannheimia haemolytica* Serotype A1 Goat Anti-Rat IgG) will be spread on 1 cm of the control line (2). It is dried by incubation at room temperature for 1 night.

The control line (2) is the line on the nitrocellulose where it is controlled whether the sample is in the sufficient amount and whether the test platform having 0,1-0,5 µg secondary antibody *(Mannheimia haemolytica* Serotype A1 Goat Anti-Rat IgG) 1 cm there on works.

### Sample Pad (3)

The sample pad (3) can be obtained from commercial manufacturer companies. It is a pad having a cellulose structure where the target analyte (antigen) is dripped onto, containing *M*. *haemolytica* Serotype A1 capsule protein. This pad is where for example, at least 100 microliters on the lateral flow test platform is loaded. The sample moves from this point to the Conjugation Pad with capillary flow force of the fluids. As an example, deep nasopharyngeal swap and bronchoalveolar lavage fluid are used.

### Waste Pad (7)

Waste Pad (7) is a pad having a cellulose structure in which the excess sample is accumulated, and it can be obtained from commercial manufacturer companies.

### Support Card (6)

The support card is a thick cardboard plate which serves as a support on which the sample pad (3), conjugation pad (4), nitrocellulose membrane (5) and waste pad (7) are adhered.

### Combining the Membrane and Pads

Nitrocellulose membrane (5) is adhered on the support card (6). The sample pad (3) is adhered on the conjugation pad (4) such that it covers 2 mm of the conjugation pad and the conjugation pad (4) is attached onto the nitrocellulose membrane (5) such that it covers 2 mm of said nitrocellulose membrane. The waste pad (7) is attached on the support card (6) and on the nitrocellulose membrane (5) such that it covers 2 mm of said nitrocellulose membrane.

### Cutting the Combined Membranes

Pad membranes that have been combined, composed of the sample pad (3), conjugation pad (4), nitrocellulose membrane (5) and waste pad (7) that are attached onto the support card (6) are cut to be 4 mm each and are placed in the cassette.

### Placement in the Cassette

The pad-membranes cut into 4 mm*7 cm size are placed in the cassette. The cassette is the protective plastic cover composed of two pieces as the bottom and top, containing the completely combined and cut pad-membranes. On the top cover of the cassette, there is an observation window which enables monitoring the test and control lines and which enables the sample to be dripped onto the sample pad. It is stored by being placed in light-proof packages containing silica gel in order to preserve it from moisture.

With the *M. haemolytica* serotype A1 lateral flow immunochromatography diagnosis kit of the invention, the antibodies are shown on the Test Line (1) as visible lines if the target analyte *(Mannheimia haemolytica* Serotype A1 capsule protein antigen) is present in the sample that is dripped (applied) onto the sample pad. If the target analyte (*Mannheimia haemolytica* Serotype A1 capsule protein antigen) is not present on the tested sample, the sample arrives at the Control Line (2) with the capillary flow force and forms a complex with the secondary antibody *(Mannheimia haemolytica* Serotype A1 Goat Anti-Rat IgG) here, indicating that the test platform works. The excess antibodies are accumulated on the waste pad. Thereby, the samples that are desired to be tested are diagnosed accurately in a brief period of time.

## Claims

1. Lateral flow immunochromatography diagnosis kit for diagnosing *Mannheimia haemolytica* bacteria **characterized in that** it comprises;
i. Nitrocellulose membrane (5)
ii. a test line (1) comprising bound monoclonal antibody produced against the *Mannheimia haemolytica* Serotype A1 capsule protein on the nitrocellulose membrane (5) and a control line (2) comprising bound secondary antibody,
iii. a sample pad (3) located on the support card (6) which serves as the base material,
iv. a conjugation pad (4) impregnated with the conjugate that comprises 10% by weight monoclonal antibody developed against *Mannheimia haemolytica* Serotype A1 capsule protein which is a different clone and recognizing a different epitope of the same antigen than the antibody used on the test line (1) and 90% by weight the marker/colorant molecules and
v. a waste pad (7).

2. Diagnosis kit according to Claim 1, **characterized in that** at least one from the group comprising colloidal gold nanoparticles, cellulose nanoparticles, latex and colloidal carbon is selected as the marker/colorant molecule.

3. Diagnosis kit according to Claim 1, **characterized in that** 1 cm of the said conjugate pad comprises 6-8 µl conjugate.

4. Diagnosis kit according to Claim 1, **characterized in that** 1 cm of said test line comprises 0,1-0,5 µg *Mannheimia haemolytica* Serotype A1 monoclonal antibody.

5. Diagnosis kit according to Claim 1, **characterized in that** 1 cm of said control line comprises 0,1-0,5 µg secondary antibody.

6. Diagnosis kit according to Claim 1, **characterized in that** the concentration of said conjugate is 0,06-0,1 mg/ml.

7. Diagnosis kit according to Claim 1, **characterized in that** the pH of said conjugate is 8-9.

8. Diagnosis kit according to Claim 1, **characterized in that** *Mannheimia haemolytica* Serotype A1 capsule protein is Rat antiserotype specific antigen 1 mAb.

9. Diagnosis kit according to Claim 1, **characterized in that** secondary antibody is Goat anti rat IgG.

## Patentansprüche

1. Lateral Flow Immunochromatographie-Diagnose kit zur Diagnose von *Mannheimia haemolytica* Bakterien, **dadurch gekennzeichnet, dass** es Folgendes umfasst:
i. Nitrozellulosemembran (5)
ii. Eine Testlinie (1), die gebundenen monoklonalen Antikörper enthält, die gegen das *Mannheimia haemolytica* Serotyp A1 Kapselprotein auf der Nitrozellulosemembran (5) gerichtet sind, und eine Kontrolllinie (2), die gebundenen sekundären Antikörper umfasst,
iii. Ein Probenpad (3), das auf der Trägerkarte (6) angeordnet ist und als Basismaterial dient,
iv. Ein Konjugationspad (4), das mit dem Konjugat imprägniert ist, das 10 % des Gewichts an monoklonalen Antikörpern enthält, die gegen das *Mannheimia haemolytica* Serotyp A1 Kapselprotein entwickelt wurden, welches ein anderer Klon ist und ein anderes Epitope des gleichen Antigens erkennt als der Antikörper, der auf der Testlinie (1) verwendet wird, und 90 Gew.- % an Marker-/Farbstoffmolekülen,
v. Ein Abfallpad (7)

2. Diagnose kit gemäß Anspruch 1, **gekennzeichnet dadurch, dass** mindestens eines aus der Gruppe bestehend aus kolloidalen Goldnanopartikeln, Cellulose-Nanopartikeln, Latex und kolloidalem Kohlenstoff als Marker/Farbstoffmolekül ausgewählt wird.

3. Diagnose kit gemäß Anspruch 1, **gekennzeichnet dadurch, dass** 1 cm des genannten Konjugatpads 6-8 µl Konjugat umfasst.

4. Diagnose kit gemäß Anspruch 1, **gekennzeichnet dadurch, dass** 1 cm der genannten Testlinie 0,1-0,5 µg *Mannheimia haemolytica* Serotyp A1 monoklonalen Antikörpers umfasst.

5. Diagnose kit gemäß Anspruch 1, **gekennzeichnet dadurch, dass** 1 cm der genannten Kontrolllinie 0,1-0,5 µg sekundären Antikörpers umfasst.

6. Diagnose kit gemäß Anspruch 1, **gekennzeichnet dadurch, dass** die Konzentration des genannten Konjugats 0,06-0,1 mg/ml beträgt.

7. Diagnose kit gemäß Anspruch 1, **gekennzeichnet dadurch, dass** der pH des genannten Konjugats 8-9 beträgt.

8. Diagnose kit gemäß Anspruch 1, **gekennzeichnet dadurch, dass** das *Mannheimia haemolytica* Serotyp A1 Kapselprotein das Rattenantiseotyp-spezifisches Antigen 1 mAb ist.

9. Diagnosekit gemäß Anspruch 1, **gekennzeichnet dadurch, dass** der sekundäre Antikörper Ziegen-anti-Ratten-IgG ist.

## Revendications

1. Kit de diagnostic d'immunochromatographie à flux latéral pour le diagnostic de la bactérie *Mannheimia haemolytica,* **caractérisé en ce qu'**il comprend :
i. une membrane de nitrocellulose (5),
ii. une ligne de test (1) comprenant l'anticorps monoclonal lié produit contre la protéine de capsule du Serotype A1 de *Mannheimia haemolytica* sur la membrane de nitrocellulose (5) et une ligne de contrôle (2) comprenant l'anticorps secondaire lié,
iii. un tampon d'échantillon (3) situé sur la carte de support (6) qui sert de matériau de base,
iv. un tampon de conjugaison (4) imprégné du conjugué qui comprend 10 % en poids d'anticorps monoclonal développé contre la protéine de capsule du sérotype A1 de *Mannheimia haemolytica,* qui est un clone différent et qui reconnaît un épitope différent du même antigène que l'anticorps utilisé sur la ligne de test (1), et 90 % en poids de molécules de marqueur/colorant et
v. un tampon de déchets (7).

2. Kit de diagnostic selon la revendication 1, **caractérisé en ce qu'**au moins un du groupe comprenant les nanoparticules d'or colloïdal, les nanoparticules de cellulose, le latex et le carbone colloïdal est choisi comme molécule marqueur/colorant.

3. Kit de diagnostic selon la revendication 1, **caractérisé en ce que** 1 cm de ladite tampon de conjugaison comprend 6-8 µl de conjugué.

4. Kit de diagnostic selon la revendication 1, **caractérisé en ce que** 1 cm de ladite ligne de test comprend 0,1-0,5 µg d'anticorps monoclonal du sérotype A1 de *Mannheimia haemolytica.*

5. Kit de diagnostic selon la revendication 1, **caractérisé en ce que** 1 cm de ladite ligne de contrôle comprend 0,1-0,5 µg d'anticorps secondaire.

6. Kit de diagnostic selon la revendication 1, **caractérisé en ce que** la concentration dudit conjugué est de 0,06-0,1 mg/ml.

7. Kit de diagnostic selon la revendication 1, **caractérisé en ce que** le pH dudit conjugué est de 8-9.

8. Kit de diagnostic selon la revendication 1, **caractérisé en ce que** la protéine de capsule du sérotype A1 de *Mannheimia haemolytica* est l'AcM 1 de l'antigène spécifique de l'antisérotype de Rat.

9. Kit de diagnostic selon la revendication 1, **caractérisé en ce que** l'anticorps secondaire est l'IgG anti-rat de Chèvre.
